# EUROPEAN PATENT SPECIFICATION

(11) **EP 3 714 853 B1**
(45) Date of publication and mention of the grant of the patent: **22.11.2023**
(21) Application number: 18881460.2
(22) Date of filing: 15.10.2018
(51) Int. Cl.: A61F 13/511, A61F 13/512

(54) **ABSORBENT ARTICLE**
ABSORBIERENDER ARTIKEL
ARTICLE ABSORBANT

(30) Priority: 24.11.2017 JP 2017226327
(43) Date of publication of application: 30.09.2020
(73) Proprietor: Daio Paper Corporation, Shikokuchuo-shi, Ehime 799-0492 (JP)
(72) Inventor: OHTAKE, Yuya, Sakura-shi Tochigi 329-1411 (JP)
(74) Representative: Williams Powell
(86) International application number: PCT/JP2018/038274
(87) International publication number: WO 2019/102744

(56) References cited:
- WO-A1-2008/146594
- WO-A1-2013/099625
- WO-A1-2017/030136
- WO-A1-2017/030136
- JP-A- S6 269 867
- JP-A- H05 228 173
- JP-A- H08 246 321
- JP-A- H08 507 451
- JP-A- 2002 105 835
- JP-A- 2003 116 909
- JP-A- 2008 073 396
- JP-A- 2015 112 187
- JP-A- 2017 153 915
- US-A1- 2017 056 256

## Description

### Technical Field

The present invention relates to an absorbent article including a disposable diaper and a sanitary napkin.

### Background Art

This type of absorbent article includes an absorber and a liquid pervious top sheet that covers a front surface side of the absorber, and excreted liquid such as urine or menses passes through the top sheet and is absorbed and held by the absorber. Conventionally, as the top sheet for the absorbent article, a nonwoven fabric formed by various manufacturing methods, the nonwoven fabric subjected to perforation as secondary processing, a perforated film made of a synthetic resin such as polyethylene, etc. have been used.

The absorbent article is required not only to prevent the excreted liquid from leaking but also prevent discomfort, rash, etc. caused by re-adhesion of the excreted liquid to a skin. In addition, it is required to prevent stuffiness before and after excretion. For this reason, in recent years, when a nonwoven fabric is used for the top sheet, for example, as shown in Patent Literatures 1 and 2, a large number of dome-shaped (hemispherical or similar convex structures) convexes are formed on the top sheet by embossing to reduce a contact area between the top sheet and the skin, thereby attempting re-adhesion of the excreted liquid and improvement of air permeability. In particular, as those described in Patent Literatures 1 and 2, a mode in which parts around the convexes (grooves between convexes) in the top sheet are pressed in a thickness direction by heating embossing and bonded to a member on a back surface side as necessary is significantly preferable since the convexes are firmly formed, and the convexes are firmly maintained even in a pressurized state in a packaging bag until use through a distribution process, an absorbing property, etc. is excellent, and appearance is excellent. The top sheet having such concaves and convexes is significantly important not only in that the top sheet has high functionality but also in that the top sheet gives consumers functional beauty. Similar disclosures are found in Patent Literatures 3 and 4.

However, when bottom portions of the grooves (concaves) surrounding the convexes are compressed in the thickness direction, a liquid pervious property of the bottom portions in the thickness direction decreases due to increase in the density of the bottom portions. Naturally, since the excreted liquid concentrates in the grooves, a decrease in the liquid pervious property in the thickness direction at the bottom portions of the grooves causes a decrease in the overall absorption rate.

### Citation List

### Patent Literature

Patent Literature 1: JP 2017-140251 A
Patent Literature 2: JP 2013-255557 A
Patent Literature 3: US 2017/0056256 A
Patent Literature 4: JP H8-246321 A

### Summary of Invention

### Technical Problem

In order to improve the liquid pervious property at the bottom portions of the grooves, there is an option not to compress the bottom portions of the grooves in the thickness direction. However, in this case, peripheral edge shapes of the convexes become unclear due to pressure during manufacturing or after manufacturing, and the appearance as functional beauty deteriorates. In addition, as described in Patent Literature 2, it is one idea to provide through-holes penetrating in the thickness direction at the bottom portions of the grooves at intervals in a continuous direction of the groove. However, a liquid pervious property of a part not having holes is not improved, it is necessary to provide a plurality of holes to ensure a sufficient liquid pervious property, and there is concern that so-called back-flow (excreted liquid moving to the back surface side of the top sheet adheres to the skin on the front surface side of the top sheet) is likely to occur.

Therefore, a main object of the invention is to provide an absorbent article including a top sheet that is excellent in maintaining shapes of the concaves and convexes and hardly causes a decrease in absorption rate.

### Solution to Problem

Absorbent articles solving the above problems are as follows.

### <First Aspect>

An absorbent article including
an absorber, and
a top sheet that covers a front surface side of the absorber,
the top sheet containing a nonwoven fabric,
a plurality of dome-shaped convexes protruding to the front surface side being arranged on the top sheet, grooves being formed between adjacent convexes,
in which through-holes penetrating the top sheet in a thickness direction are provided at intervals in a circumferential direction of each of the convexes, the through-holes having front and back sides,
peripheral edge portions of the through-holes are high-density portions where constituent fibers of the nonwoven fabric are pushed to parts around the through-holes from positions corresponding to the through-holes,
the high-density portions are not compressed in the thickness direction,
the peripheral edge portions of the through-holes rise to both the front and back sides,
a part of peripheral edges of the convexes is formed by a part of the peripheral edge portions of the through-holes, and
a difference between a maximum value and a minimum value of an apparent thickness is in a range of 0 to 50% of the maximum value in a portion of the top sheet excluding the peripheral edge portions of the through-holes.

### (Function and Effect)

In this absorbent article, the difference between the maximum value and the minimum value of the apparent thickness is in a range of 0 to 50% of the maximum value in the portion of the top sheet excluding the peripheral edge portions of the through-holes, and bottom portions of the grooves have a similar density to that of the convexes. Thus, the liquid pervious property in the thickness direction at the bottom portions of the grooves is similar to that in the convexes, etc., and the liquid pervious property in the thickness direction at the bottom portions of the grooves is not low as in the conventional example in which the bottom portions of the grooves are compressed in the thickness direction. In addition, the peripheral edge portions of the through-holes become high-density portions, correspond to barrel shaped portions where the constituent fibers of the nonwoven fabric are pushed to parts around the through-holes from the positions corresponding to the through-holes, and are not compressed in the thickness direction. Such high-density portions are extremely thin in a direction orthogonal to the thickness direction, and hardly affect the liquid pervious property in the thickness direction. Therefore, even when the excreted liquid concentrates in the grooves, the fact that there is almost no decrease in the liquid pervious property in the thickness direction at the bottom portions of the grooves and the fact that the through-holes significantly improve the liquid pervious property are combined so that the overall absorption rate is less likely to decrease. In addition, a part of the peripheral edges of the convexes are formed by a part of the high-density portions (the peripheral edge portions of the through-holes). As a result, the peripheral edges of the convexes are reinforced by the high-density portions, and peripheral edge shapes of the convexes are less likely to collapse, that is, the convexes are excellent in a shape maintaining property. In this aspect, the liquid pervious property of the bottom portions of the grooves may be high, and a main function of the through-holes can correspond to a function for maintaining the shapes of the convexes.

### <Second Aspect>

The absorbent article according to the first aspect,
in which a fineness of fibers of the top sheet is in a range of 1.0 to 3.0 dtex, and
a fiber density of the portion of the top sheet excluding the peripheral edge portions of the through-holes is in a range of 16 to 32 g/m³.

### (Function and Effect)

The above-described structure of the top sheet is suitable for the nonwoven fabric having the fineness and density of this aspect.

### <Third Aspect>

The absorbent article according to the first or second aspect,
in which the convexes have staggered arrangement,
the through-holes are disposed on both sides in a front-back direction at a center of each of the convexes in a width direction and both sides in the width direction at a center thereof in the front-back direction, and each of the peripheral edge portions of the through-holes forms parts of both peripheral edges of adjacent convexes, and
each of the grooves extends between adjacent through-holes in a direction along between adjacent convexes.

### (Function and Effect)

From a viewpoint of the back-flow, it is preferable that the number of through-holes is small. However, as the number of through-holes decreases, the shape maintaining property of the convexes deteriorates. Therefore, it is preferable that the convexes and the through-holes are arranged as in this aspect from a viewpoint of achieving both back-flow and the shape maintaining property of the convexes.

### <Fourth Aspect>

The absorbent article according to any one of the first to third aspects, in which each of the peripheral edge portions of the through-holes has a weir-shaped portion expanding at least on the front surface side.

### (Function and Effect)

In a case where the through-holes are formed by the needle piercing process, when the constituent fibers of the nonwoven fabric are pushed to parts around the through-hole from the positions corresponding to the through-holes, the peripheral edge portions of the through-holes rise (bulge or warp) to both the front and back sides by the fibers escaping to both the front and back sides along a side surface of a needle. This rising functions as a weir against an inflow of the liquid to the through-holes even when either one is located on the front surface side of the top sheet. Therefore, in the case of having such a weir-shaped portion, after the absorption proceeds and a liquid level in the grooves becomes lower than that of the weir-shaped portion, the liquid pervious property at the bottom portions of the grooves determines the absorption rate. Therefore, the structure of the top sheet described above is particularly effective when the weir-shaped portion is provided as in this aspect.

### <Fifth Aspect>

The absorbent article according to any one of the first to fourth aspects,
in which a dimension of each of the convexes in the front-back direction is in a range of 3 to 10 mm and a dimension thereof in the width direction is in a range of 3 to 10 mm, and
a dimension of each of the through-holes in the front-back direction is in a range of 15 to 35% of a dimension of each of the convexes in the front-back direction, and a dimension of each of the through-holes in the width direction is in a range of 15 to 35% of a dimension of each of the convexes in the width direction.

### (Function and Effect)

In the above-described structure of the top sheet, it is preferable that the dimensions of the convexes and the through-holes are within the ranges of this aspect.

### Advantageous Effects of Invention

As described above, according to the invention, it is advantageous in that a shape maintaining property of the concaves and convexes is excellent, and a decrease in the absorption rate is less likely to occur.

### Brief Description of Drawings

Fig. 1 is a plan view illustrating an internal surface side of a pad-type disposable diaper in a spread state.
Fig. 2 is a plan view illustrating only a main part.
Fig. 3 is a cross-sectional view taken along line Y-Y of Fig. 1.
Fig. 4 is a cross-sectional view taken along line X-X of Fig. 1.
Fig. 5(a) is an enlarged plane view of a main part in a spread state, Fig. 5(b) is a cross-sectional view taken along line 2-2, and Fig. 5(c) is a cross-sectional view taken along line 3-3.
Fig. 6 is a cross-sectional view corresponding to an X-X cross section of Fig. 1.
Fig. 7 is a cross-sectional view schematically illustrating an absorption mechanism of an absorber.
Fig. 8 is a cross-sectional view schematically illustrating an absorption mechanism of the absorber.
Fig. 9 is a plan view of another absorber.
Fig. 10 is an enlarged plan view of a main part in a spread state.
Fig. 11 is an enlarged plan view of a main part in a spread state.
Fig. 12 is an enlarged plan view of a main part of a top sheet.

### Description of Embodiments

Hereinafter, examples of an absorbent article will be described in detail with reference to the accompanying drawings. Note that a term "crotch portion" refers to a part corresponding to a crotch of a body during use, and may correspond to a range from a center or the vicinity thereof of the article in a front-back direction LD to a predetermined site on a front side as in the illustrated embodiment, or a predetermined range at the center of the article in the front-back direction LD depending on the product. In the case of having narrowing portions having a narrow width in the middle of the article in the front-back direction LD or in the middle of the absorber in the front-back direction LD, the term refers to a predetermined front-back direction range in which a minimum width site of one or both of the narrowing portions corresponds to a center in the front-back direction. In addition, a "front side portion (ventral side portion)" refers to a portion on the front side of the crotch portion, and a "back side portion (dorsal side portion)" refers to a portion on the back side of the crotch portion. In addition, a dotted pattern portion in the cross-sectional views indicates an adhesive as bonding means that bonds respective components located on the front surface side and the back surface side thereof, and is formed by solid, bead, curtain, summit, or spiral coating of a hot melt adhesive, or pattern coating (transfer of the hot melt adhesive in a letterpress method), or application of an elastic member to an outer peripheral surface such as comb gun or sure wrap application instead of or together with the above methods in a fixed part of the elastic member. Examples of the hot melt adhesive include EVA-based, pressure sensitive adhesion rubber-based (elastomer-based), polyolefin-based, and polyester/polyamide-based adhesives, and can be used without any particular limitation. As bonding means that bonds respective components, it is possible to use means by material welding such as heat sealing or ultrasonic sealing.

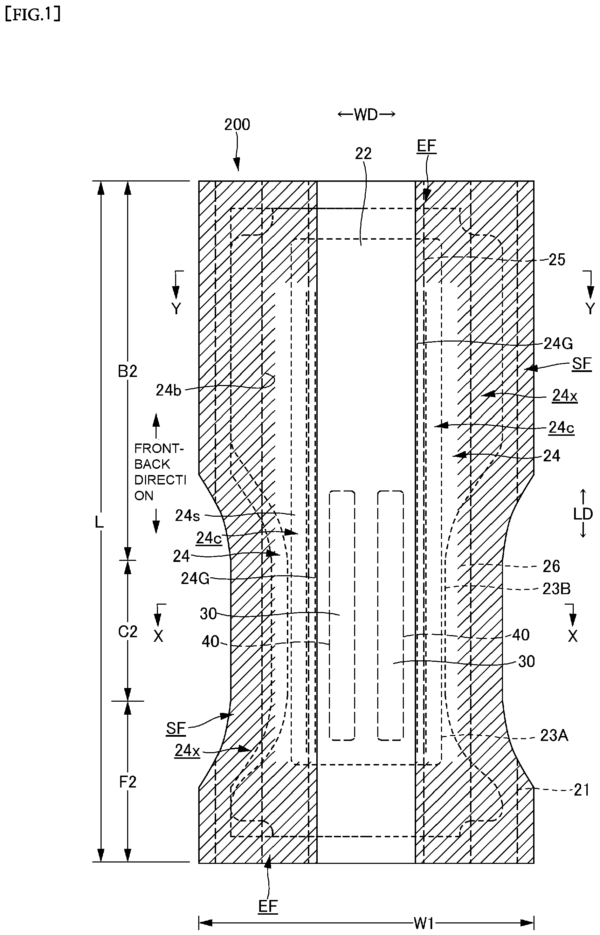

Fig. 1 to Fig. 4 illustrate a pad-type disposable diaper 200 as an example of the absorbent article. The pad-type disposable diaper 200 has a crotch portion C2, and a front side portion F2 and a back side portion B2 extending on both front and back sides thereof. The dimensions of each portion can be determined as appropriate. For example, a maximum length of the article (length in the front-back direction) L can be set to about 350 to 700 mm, and a maximum width W1 can be set to about 130 to 400 mm (however, wider than an absorption surface of the diaper). In this case, a length of the crotch portion C2 in the front-back direction can be set to about 10 to 150 mm, a length of the front side portion F2 in the front-back direction can be set to about 50 to 350 mm, and a length of the back side portion B2 in the front-back direction can be set to about 50 to 350 mm. In addition, a width W3 of the crotch portion C2 can be set to 150 mm or more, particularly about 200 to 260 mm for an adult.

The pad-type disposable diaper 200 has a basic structure in which absorbers 23A and 23B are interposed between a liquid impervious sheet 21 and a liquid pervious top sheet 22.

On the back surface side of the absorbers 23A and 23B, the liquid impervious sheet 21 is provided so as to slightly protrude from peripheral edges of the absorbers 23A and 23B. As the liquid impervious sheet 21, in addition to a polyethylene film, etc., it is possible to use a sheet having moisture permeability without impairing a water blocking property from a viewpoint of preventing stuffiness. As this water blocking and moisture-permeable sheet, for example, it is possible to use a microporous sheet obtained by melt kneading an inorganic filler in a polyolefin resin such as polyethylene or polypropylene to form a sheet, and then monoaxially or biaxially stretching the sheet.

Further, an outer surface of the liquid impervious sheet 21 is covered with an outer sheet 27 made of a nonwoven fabric, and the outer sheet 27 protrudes outside peripheral edges of the liquid impervious sheet 21 with a predetermined width. Various nonwoven fabrics can be used as the outer sheet 27. As a material fiber included in the nonwoven fabric, in addition to a synthetic fiber such as a polyolefin-based fiber such as polyethylene or polypropylene, a polyester-based fiber, or a polyamidebased fiber, it is possible to use a regenerated fiber such as rayon and cupra, and a natural fiber such as cotton. The outer sheet 27 can be omitted.

The front surface sides of the absorbers 23A and 23B are covered with the top sheet 22 having the liquid pervious property. In the illustrated embodiment, the absorbers 23A and 23B partially protrude from side edges of the top sheet 22. However, the width of the top sheet 22 can be increased so that side edges of the absorbers 23A and 23B do not protrude. Various nonwoven fabrics can be used as the top sheet 22. A constituent fiber of the nonwoven fabric may be a short fiber or a long fiber (continuous fiber). In addition to a synthetic fiber such as a polyolefin-based fiber such as polyethylene or polypropylene, a polyester-based fiber, or a polyamide-based fiber (which may correspond to a single-component fiber or a composite fiber of a core-sheath structure, etc. including a plurality of components), it is possible to use a regenerated fiber such as rayon and cupra, and a natural fiber such as cotton. In addition, a method for binding fibers of the nonwoven fabric is not particularly limited, and may correspond to chemical means such as an adhesive or a solvent, or physical means such as mechanical entanglement or thermal bonding. One of preferable nonwoven fabrics as the top sheet is an air through nonwoven fabric obtained by bonding thermoplastic synthetic fibers with hot air. Details of the top sheet 22 will be described later.

It is desirable to interpose an intermediate sheet 25 between the top sheet 22 and the absorbers 23A and 23B. The intermediate sheet 25 is provided to prevent back-flow of urine absorbed by the absorbers 23A and 23B, and it is desirable to use a material having low water retention and high liquid pervious property, such as various nonwoven fabrics and mesh films. When a front end of the top sheet 22 is set to 0% and a back end of the top sheet 22 is set to 100%, a front end of the intermediate sheet 25 is preferably located in a range of 0 to 11%, and a back end of the intermediate sheet 25 is preferably located in a range of 92 to 100%. In addition, a width W4 of the intermediate sheet 25 is preferably about 50 to 100% of a minimum width W5 of a narrowing portion 23n of the absorbers 23A and 23B described later. The intermediate sheet 25 can be omitted.

At both end portions of the pad-type disposable diaper 200 in the front-back direction LD, end flap portions EF are formed in which the outer sheet 27 and the top sheet 22 are extended and bonded to both front and back sides of front and back ends of the absorbers 23A and 23B, respectively, and the absorbers 23A and 23B are not present. At both side portions of the pad-type disposable diaper 200, the outer sheet 27 extends outside the side edges of the absorbers 23A and 23B, and on an inner surface of a portion from this extending portion to a side portion of the top sheet 22, outer portions 24x of gather sheets 24s forming a three-dimensional gather 24 in a width direction WD are attached over the entire part in the front-back direction LD to form side flap portions SF in which the absorbers 23A and 23B are not present. These bonded portions are indicated by hatched patterns in Fig. 1, and can be formed by hot melt adhesive, heat seal, and ultrasonic seal. When the outer sheet 27 is not provided, the liquid impervious sheet 21 may be extended to the side flap portions SF instead of the outer sheet 27 to form external surface sides of the side flap portions SF.

As a material of the gather sheets 24s, a plastic sheet or a melt-blown nonwoven fabric can be used. However, from a viewpoint of feel to the skin, a nonwoven fabric subjected to a water-repellent treatment with silicone, etc. is preferably used.

Portions 24c on center sides of the gather sheets 24s in the width direction extend up to the top sheet 22, and an elongated elastic member 24G is fixed to an end portion on the center side in the width direction by the hot melt adhesive, etc. in a stretched state along the front-back direction. Examples of the elongated elastic member 24G include a commonly used material such as polystyrene-based rubber, polyolefin-based rubber, polyurethane-based rubber, polyester-based rubber, polyurethane, polyethylene, polystyrene, styrene-butadiene copolymer, silicone, or polyester formed in a thread shape, a string shape, a band shape, etc.

In addition, in both the gather sheets 24s, the outer portions 24x in the width direction are bonded and fixed to an inner surface of the article (in the illustrated embodiment, a front surface of the top sheet 22 and an inner surface of the outer sheet 27) over the entire part in the front-back direction, and the portions 24c on the center sides in the width direction are bonded and fixed to the inner surface of the article (in the illustrated embodiment, the front surface of the top sheet 22) at both end portions in the front-back direction and are not fixed to the inner surface of the article (in the illustrated embodiment, the front surface of the top sheet 22) between both the end portions in the front-back direction. As illustrated in Fig. 1, this non-fixed portion is a portion serving as a leakage prevention wall that elastically stands with respect to the inner surface of the article (in the illustrated embodiment, the front surface of the top sheet 22), and standing base edges 24b are located at boundaries between the fixed portions 24x on the outer sides and the portions 24c on the inner sides in the width direction in the gather sheets 24s.

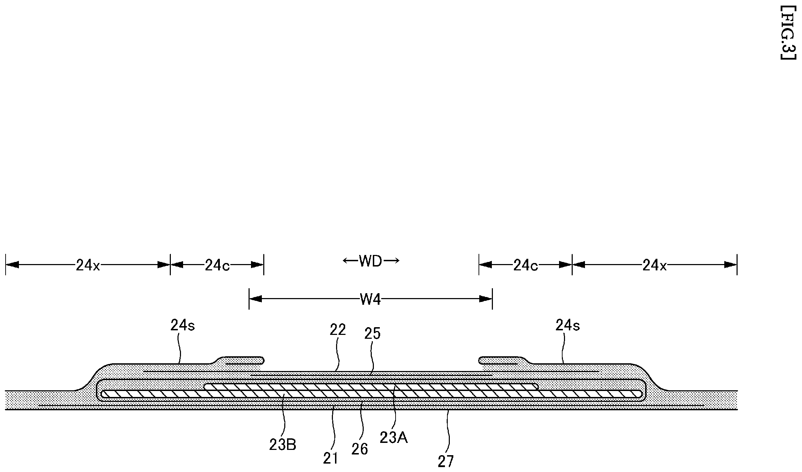

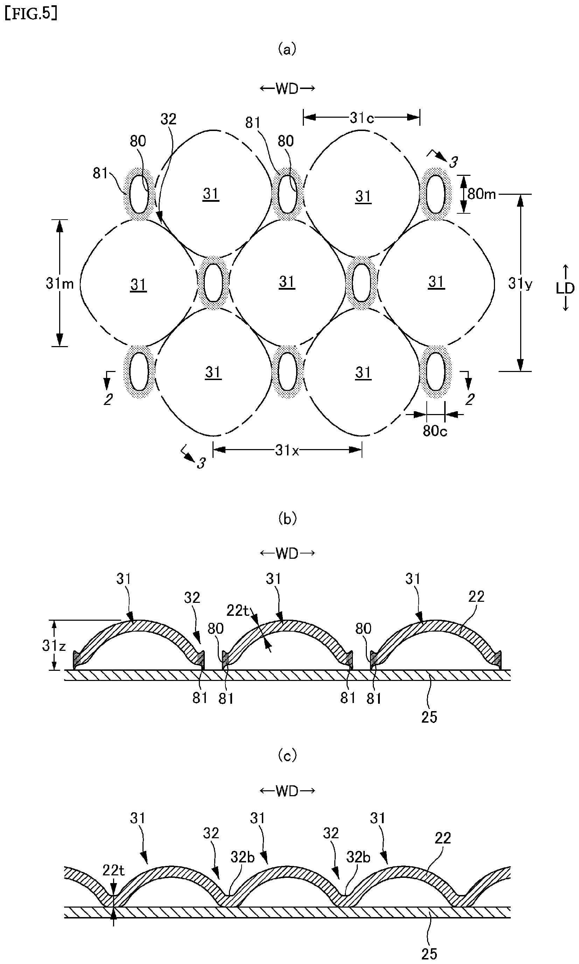

As the absorbers 23A and 23B, it is possible to use basically an accumulated body of pulp fibers 23f, an assembly of filaments such as cellulose acetate, or a nonwoven fabric, and as necessary, a material obtained by mixing and fixing super absorbent polymer particles 23p in the form of particles therewith, etc. As illustrated in Fig. 3 and Fig. 5, the absorbers 23A and 23B may have a single-layered structure in addition to a two-layered structure including the lower layer absorber 23B and the upper layer absorber 23A provided on the front surface side thereof. When the absorbers 23A and 23B have the two-layered structure, the lower layer absorber 23B is preferably an aggregate of at least the pulp fibers 23f, and particularly preferably a mixed aggregate of the pulp fibers 23f and the super absorbent polymer particles 23p. On the other hand, the upper layer absorber 23A is preferably a mixed aggregate of the pulp fibers 23f and the super absorbent polymer particles 23p.

As the super absorbent polymer particles 23p contained in the upper layer absorber 23A and the lower layer absorber 23B, it is possible to use those used for this type of absorbent article without change. For example, in the case of using super absorbent polymer particles having the same particle size distribution for the upper layer absorber 23A and the lower layer absorber 23B, etc., in a normal case, it is desirable to use those in which when sieving (shaking for 5 minutes) using a standard sieve (JIS Z8801-1:2006) of 500 um and sieving (shaking for 5 minutes) using a standard sieve (JIS Z8801-1:2006) of 180 um for particles falling under the former sieve are performed, a ratio of particles remaining on the standard sieve of 500 um is 30% by weight or less, and a ratio of particles remaining on the standard sieve of 180 um is 60% by weight or more. In addition, in the case of using super absorbent polymer particles having the different particle size distributions for the upper layer absorber 23A and the lower layer absorber 23B, it is desirable that in the particle size distribution of the super absorbent polymer particles used for the upper layer absorber 23A, when sieving using the standard sieves of 500 µm and 180 µm is performed, a ratio of particles remaining on the standard sieve of 500 um is 50% by weight or less, and a ratio of particles remaining on the standard sieve of 180 um is 50% by weight or more, and it is desirable that in the particle size distribution of the super absorbent polymer particles used for the lower layer absorber 23B, when sieving using the standard sieves of 500 um and 180 um is performed, a ratio of particles remaining on the standard sieve of 500 um is 25% by weight or less, and a ratio of particles remaining on the standard sieve of 180 um is 70% by weight or more.

The super absorbent polymer particles 23p are not particularly limited. However, it is possible to suitably use those having a water absorption rate of 20 to 50 seconds and a water absorption capacity of 50 to 80 g/g. Examples of the super absorbent polymer particles 23p include starch-based, cellulose-based, and synthetic polymer-based, and it is possible to use starch-polyacrylic acid (salt) graft copolymers, saponified starchacrylonitrile copolymers, sodium carboxymethyl cellulose crosslinked products, polyacrylic acid (salt) polymers, etc.

The upper layer absorber 23A and the lower layer absorber 23B can be integrally or individually wrapped with a wrapping sheet 26 such as crepe paper having the liquid pervious property and liquid holding property to hold the shape and the super absorbent polymer particles 23p as necessary.

The absorbers 23A and 23B extend from the front side portion F2 to the back side portion B2. The upper layer absorber 23A can have the same dimensions as those of the lower layer absorber 23B. However, it is desirable that the maximum length and maximum width of the upper layer absorber 23A are shorter than those of the lower layer absorber 23B as in the illustrated embodiment. In a normal case, the maximum length of the upper layer absorber 23A can be set to about 60 to 90% of the maximum length of the lower layer absorber 23B, and the maximum width of the upper layer absorber 23A can be set to about 60 to 90% of the maximum width of the lower layer absorber 23B.

Shapes of the upper layer absorber 23A and the lower layer absorber 23B can be determined as appropriate, and each of the shapes can be set to a rectangular shape. However, it is preferable that in at least the large one of the absorbers 23A and 23B (the lower layer absorber 23B in the illustrated example), a predetermined portion in the middle in the front-back direction including the crotch portion C2 is formed as the narrowing portion 23n having a narrow width. It is preferable that the minimum width W5 of the narrowing portion 23n is about 50 to 65% of a width W2 of non-narrowing portions located before and after the narrowing portion 23n. In addition, when the front end of the article is set to 0% and the back end of the article is set to 100%, the front end of the narrowing portion 23n is preferably located in a range of 10 to 25%, the back end of the narrowing portion 23n is preferably located in a range of 40 to 65%, and a site (minimum width site) of the narrowing portion 23n corresponding to the minimum width W5 is preferably located in a range of 25 to 30%.

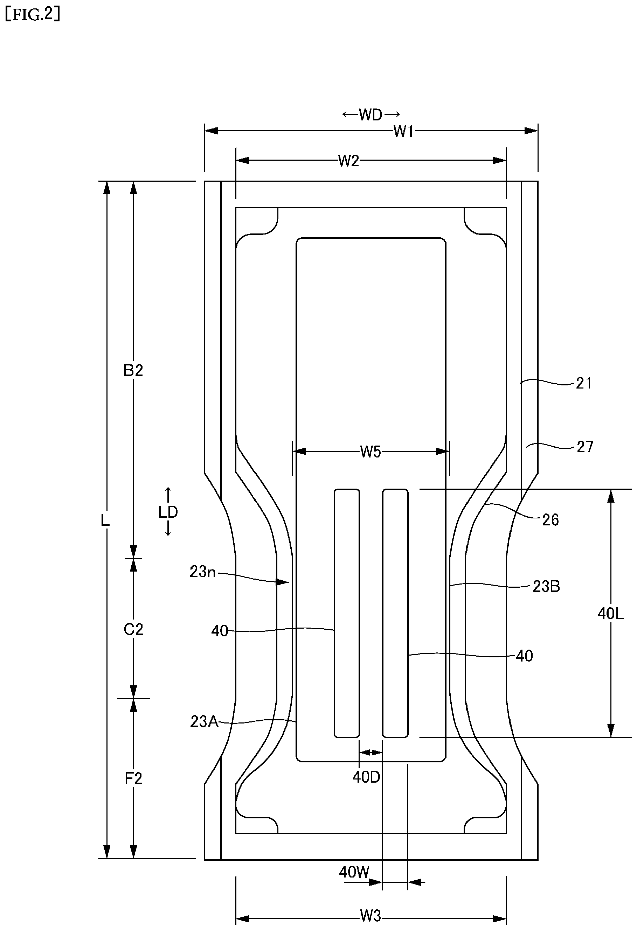

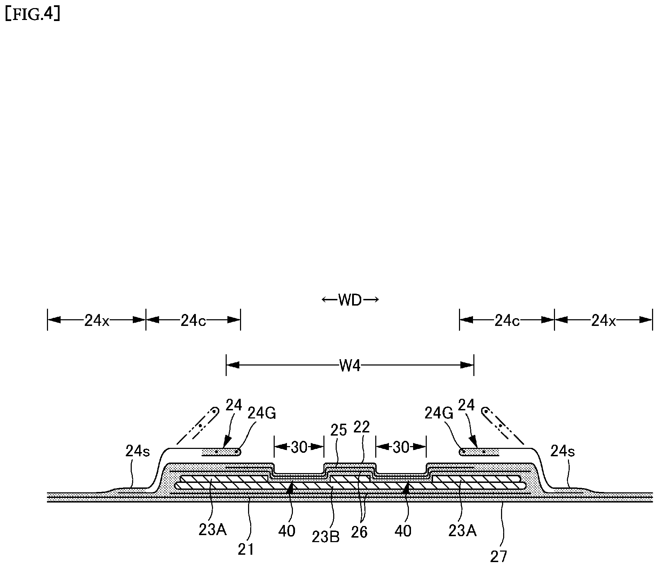

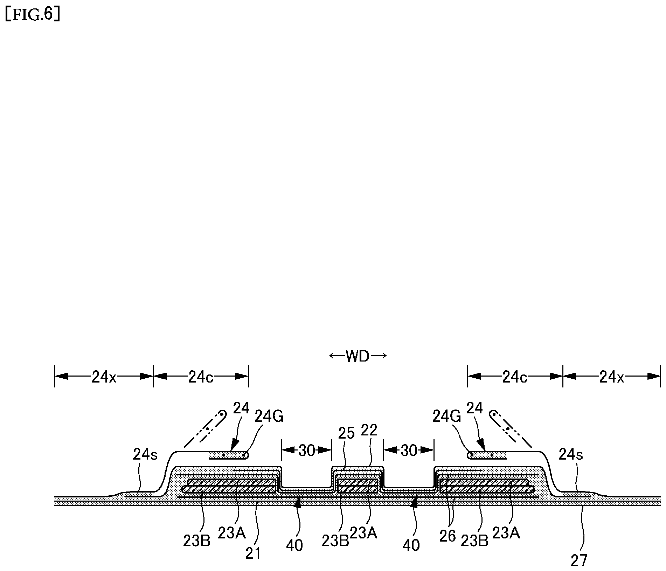

As illustrated in Fig. 1 and Fig. 2, it is preferable that a pair of right and left slits 40 having a predetermined width extends in the front-back direction only in the upper layer absorber 23A at least in a front-back direction region corresponding to the crotch portion C2. As illustrated in Fig. 4, it is possible to adopt a mode in which the slits 40 having the predetermined width are not provided in the lower layer absorber 23B (in this case, the lower layer absorber 23B may have a break having no width). In addition, as illustrated in Fig. 6, it is possible to form integral slits 40 penetrating the upper layer absorber 23A and the lower layer absorber 23B in the thickness direction. In the case of having such slits 40, the top sheet 22 may have recessed portions 30 recessed into the slits 40 as in the illustrated example, or may be hardly recessed in the slits 40.

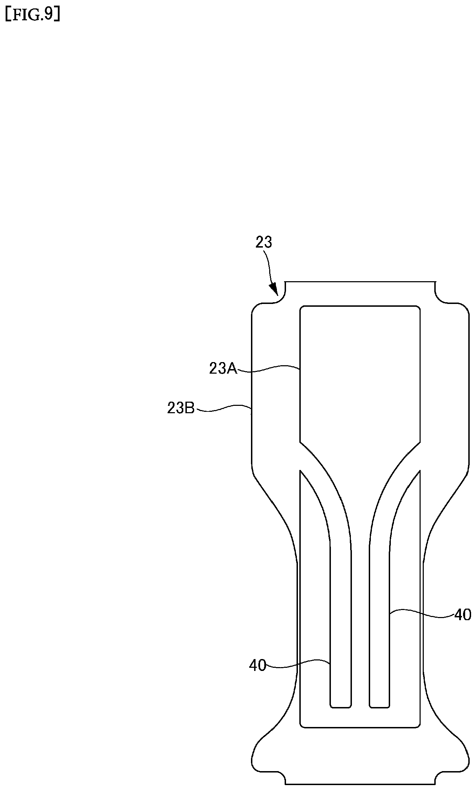

A length 40L of the slits 40 in the front-back direction is not particularly limited. Therefore, the slits 40 may be provided over the entire part of the upper layer absorber 23A in the front-back direction. However, as in the illustrated embodiment, it is desirable that the slits 40 are extended from a crotch side end portion of the front side portion F2 to a crotch side end portion of the back side portion B2. In addition, as illustrated in Fig. 9, back side portions of the slits 40 may be bent outward in the width direction (the front sides can be similarly bent). More specifically, when a front end of the disposable diaper 200 is set to 0%, and a back end of the disposable diaper 200 is set to 100%, front ends of the slits 40 are preferably located in a range of 15 to 30%, and back ends of the slits 40 are preferably located in a range of 40 to 70%.

In the upper layer absorber 23A in the illustrated embodiment, the front and back ends of the slits 40 do not penetrate peripheral edges of the upper layer absorber 23A. However, as in the example illustrated in Fig. 9, the back ends (or front ends or both ends) may reach the peripheral edges. Note that in a mode in which both the front and back ends of the slits 40 reach the side edges of the upper layer absorber 23A, portions lateral to the slits 40 are separated from a portion between the slits 40.

The slits 40 are preferably provided such that one slit is provided on each of right and left sides with an interval in the width direction. However, only one slit may be provided at a center in the width direction WD. In this case, it is preferable that a position of the slit 40 in the width direction WD is symmetrical. The pad-type disposable diaper generally has a mode in which the width of the absorbers 23A and 23B is wider than a crotch width of the wearer, both end portions of the crotch portion in the width direction correspond to portions facing inner thighs of the wearer, and an intermediate portion in the width direction corresponds to a portion facing the crotch, and thus it is desirable to provide the slits along boundaries of these portions. For this reason, it is preferable that an interval 40D between the slits 40 is usually about 10 to 30% of the minimum width W5 of the narrowing portion 23n of the absorbers 23A and 23B.

A width 40W of the slits 40 is not particularly limited as long as the side walls facing each other are separated from each other. However, in a normal case, it is desirable that the width 40W is set to about 10 to 20% of the minimum width W5 of the narrowing portion 23n of the absorbers 23A and 23B. Specifically, for an adult product, the width 40W can be set to about 5 to 32 mm.

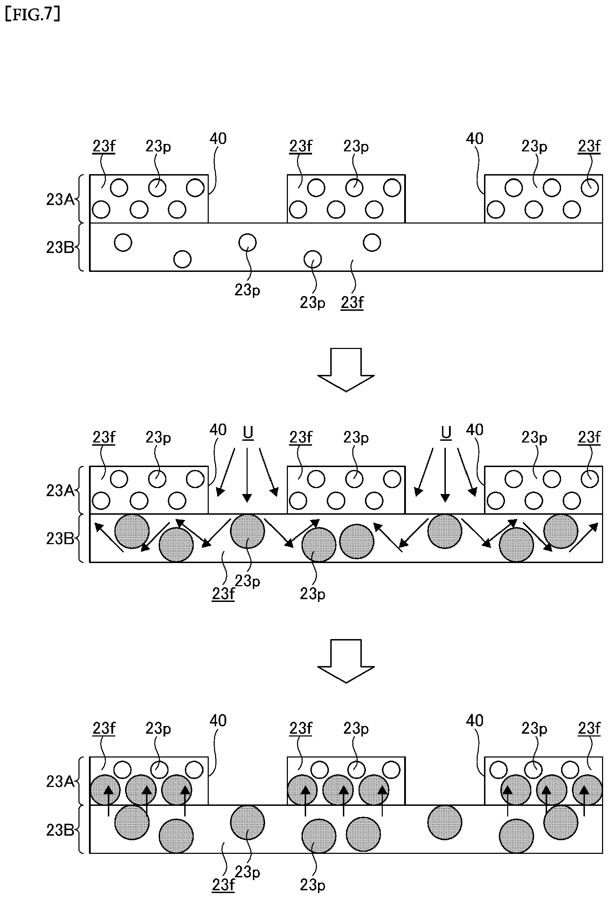

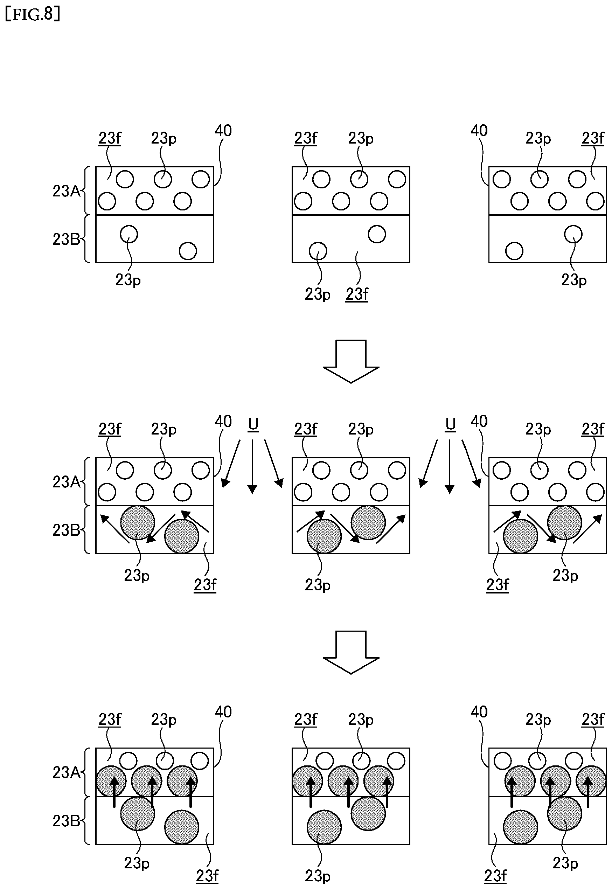

In addition, as illustrated in Fig. 7 and Fig. 8, it is preferable that a weight ratio of the super absorbent polymer particles 23p to the pulp fibers 23f in the upper layer absorber 23A is higher than a weight ratio of the super absorbent polymer particles 23p to the pulp fibers 23f in the lower layer absorber 23B. That is, in the absorbers 23A and 23B, as Fig. 7 and Fig. 8 illustrate changes of absorption states, respectively, an excrement liquid U is supplied to the upper layer absorber 23A, and is preferentially supplied to the lower layer absorber 23B through the slits 40. Here, when the weight ratio of the super absorbent polymer particles 23p to the pulp fibers 23f in the lower layer absorber 23B is lower than that of the upper layer absorber 23A, gel blocking is less likely to occur than in the upper layer absorber 23A, and the excrement liquid spreads more widely in the lower layer absorber 23B. In the figure, movement of the liquid is indicated by arrows. Then, the liquid absorbed by the lower layer absorber 23B is transferred to the upper layer absorber 23A so as to be sucked up by the upper layer absorber 23A at least after absorption saturation of the lower layer absorber 23B, and is absorbed by the upper layer absorber 23A and retained. In this instance, in the upper layer absorber 23A, the weight ratio of the super absorbent polymer particles 23p to the pulp fibers 23f is high, and a larger amount of liquid can be absorbed and retained. Further, the lower layer absorber 23B preferentially performs absorption. Thus, absorption capacity is left on the front surface side (skin side) of the upper layer absorber 23A until the end. As a result, a back-flow prevention property is further improved. Note that naturally, in Fig. 7 and Fig. 8, the sizes of the super absorbent polymer particles 23p are exaggerated for easy understanding of a content ratio and an absorption expansion change of the super absorbent polymer particles 23p.

Considering such an absorption mechanism, it is preferable that the super absorbent polymer particles 23p contained in the lower layer absorber 23B are excellent in liquid pervious property, specifically an absorption rate is in a range of 20 to 35 seconds and an absorption amount is in a range of 50 to 70 g/g, and it is suitable that the super absorbent polymer particles 23p contained in the upper layer absorber 23A have a large absorption amount, specifically an absorption rate is in a range of 60 to 80 seconds and an absorption amount is in a range of 50 to 80 g/g.

In addition, when the weight ratio of the super absorbent polymer particles 23p to the pulp fibers 23f in the upper layer absorber 23A is set higher than the weight ratio of the super absorbent polymer particles 23p to the pulp fibers 23f in the lower layer absorber 23B, the weight ratios of the super absorbent polymer particles 23p and 23p to the pulp fibers 23f in the upper layer absorber 23A and the lower layer absorber 23B can be determined as appropriate. However, when a total basis weight (sum of pulps 19f and the super absorbent polymer particles 23p) of the upper layer absorber 23A is in a range of 350 to 700 g/m², the weight ratio of the super absorbent polymer particles 23p to the pulp fibers 23f in the upper layer absorber 23A is preferably about 55 to 100%, particularly preferably 65 to 90%. In addition, when a total basis weight (sum of pulps 18f and the super absorbent polymer particles 23p) of the lower layer absorber 23B is in a range of 250 to 450 g/m², the weight ratio of the super absorbent polymer particles 23p to the pulp fibers 23f in the lower layer absorber 23B is preferably about 0 to 50%, particularly preferably 30 to 40%.

As illustrated in Fig. 5, in the top sheet 22, a plurality of dome-shaped convexes 31 protruding to the front surface side is arranged, a groove 32 is formed between adjacent convexes 31, and through-holes 80 penetrating the top sheet 22 in the thickness direction are provided at intervals in a circumferential direction of the convex 31. In addition, as illustrated in Fig. 5, peripheral edge portions 81 of the through-holes 80 are high-density portions where constituent fibers of the nonwoven fabric are pushed to parts around the through-holes 80 from positions corresponding to the through-holes 80. In addition, a part of the peripheral edges of the convexes 31 are formed by a part of the peripheral edge portions 81 of the through-holes 80. Furthermore, a difference between a maximum value and a minimum value of the apparent thickness 22t is in a range of 0 to 50% of the maximum value in a portion of the top sheet 22 excluding the peripheral edge portions 81 of the through-holes 80. The difference between the maximum value and the minimum value of the apparent thickness 22t is preferably 0 to 40% of the maximum value, more preferably 0 to 30%, and particularly preferably 0 to 20%. As described above, when the difference between the maximum value and the minimum value of the apparent thickness 22t is small in the portion of the top sheet 22 excluding the peripheral edge portions 81 of the through-holes 80, bottom portions 32b of the grooves 32 have a similar density to that of the convexes 31. Thus, the liquid pervious property in the thickness direction at the bottom portions 32b of the grooves 32 is similar to that in the convexes 31, etc., and the liquid pervious property in the thickness direction at the bottom portions 32b of the grooves 32 is not low as in the conventional example in which the bottom portions 32b of the grooves 32 are compressed in the thickness direction. In addition, the peripheral edge portions 81 of the through-holes 80 become high-density portions, correspond to barrel shaped portions where the constituent fibers of the nonwoven fabric are pushed to parts around the through-holes 80 from the positions corresponding to the through-holes 80, and are not compressed in the thickness direction. Such high-density portions are extremely thin in a direction orthogonal to the thickness direction (for example, about 1 to 3 mm in apparent thickness), and hardly affect the liquid pervious property in the thickness direction. Therefore, even when the excreted liquid concentrates in the grooves 32, the fact that there is almost no decrease in the liquid pervious property in the thickness direction at the bottom portions 32b of the grooves 32 and the fact that the through-holes 80 significantly improve the liquid pervious property are combined so that the overall absorption rate is less likely to decrease. In addition, a part of the peripheral edges of the convexes 31 are formed by a part of the high-density portions (the peripheral edge portions 81 of the through-holes 80). As a result, the peripheral edges of the convexes 31 are reinforced by the hard compressed high-density portions, and peripheral edge shapes of the convexes 31 are less likely to collapse, that is, the convexes 31 are excellent in a shape maintaining property. In this aspect, since the liquid pervious property of the bottom portions 32b of the grooves 32 may be high, it can be said that a main function of the through-holes 80 is a function for maintaining the shapes of the convexes 31.

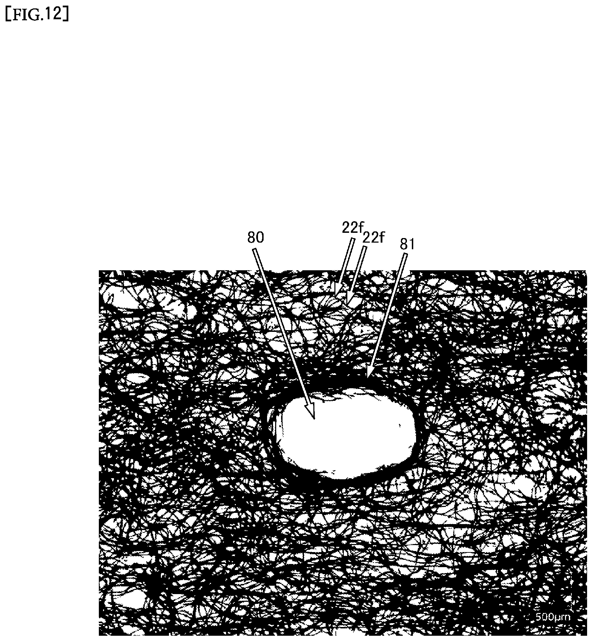

The dome-shaped convexes 31 and the grooves 32 can be formed by extruding the top sheet 22 from the back surface side to the front surface side using embossing (not sandwiching the top sheet 22 from both sides in the thickness direction). In addition, the through-holes 80 can be formed by a needle piercing process. In this case, as illustrated in Fig. 12, at the peripheral edge portions 81 of the through-holes 80, constituent fibers 22f of the nonwoven fabric are merely pushed to parts around the through-holes 80 from the positions corresponding to the through-holes 80 and not compressed in the thickness direction. Therefore, a thickness 81t greater than or equal to a thickness 22t of other portions is left at the peripheral edge portions 81 of the through-holes 80. In particular, in a case where the through-holes 80 are formed by the needle piercing process, when the constituent fibers of the nonwoven fabric are pushed to parts around the through-holes 80 from the positions corresponding to the through-holes 80, the peripheral edge portions 81 of the through-holes 80 rise (bulge or warp) to both the front and back sides by the fibers escaping to both the front and back sides along a side surface of a needle. This rising is small on an entry side of the needle and large on an opposite side in the top sheet 22 and functions as a weir against an inflow of the liquid to the through-holes 80 even when either one is located on the front surface side of the top sheet 22. Therefore, in the case of having such a weir-shaped portion, after the absorption proceeds and a liquid level in the grooves 32 becomes lower than that of the weir-shaped portion, the liquid pervious property at the bottom portions 32b of the grooves 32 determines the absorption rate. However, as described above, when the liquid pervious property of the bottom portions 32b of the grooves 32 is high, an overall influence on the absorption rate is small.

Considering flexibility and texture of the top sheet 22, the liquid pervious property of the bottom portions 32b of the grooves 32, the shape maintaining property of the peripheral edges of the convexes 31, etc. comprehensively, the fineness of the fibers of the top sheet 22 is preferably in a range of 1.0 to 3.0 dtex, particularly preferably in a range of 1.5 to 2.5 dtex, and the fiber density of the portion of the top sheet 22 excluding the peripheral edge portions 81 of the through-holes 80 is preferably in a range of 16 to 32 g/m³. Further, an air through nonwoven fabric is suitable for the top sheet 22. The top sheet 22 may be formed of a nonwoven fabric in which at least one of fineness and hydrophilicity changes in the thickness direction, for example, a nonwoven fabric in which a plurality of layers different in at least one of fineness and hydrophilicity is stacked.

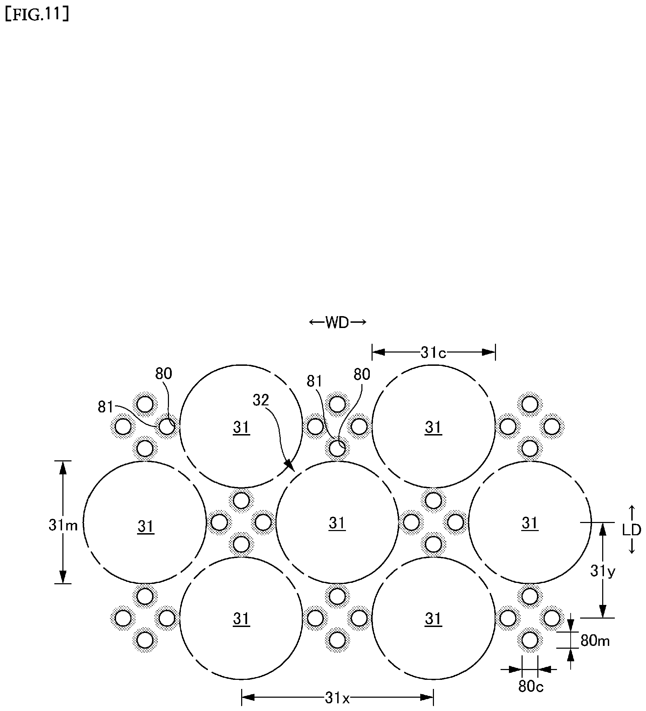

The arrangement of the convexes 31 and the arrangement and number of the through-holes 80 are not particularly limited. It is preferable that three or more, particularly four or more, of the through-holes 80 are provided at equal intervals in the circumferential direction of the convex 31. As long as a part of the peripheral edge portions 81 forms a part of the peripheral edges of the convexes 31 (including both a case where the peripheral edge portions 81 of the through-holes 80 and the peripheral edges of the convexes 31 are in contact with each other and a case where the peripheral edge portions 81 and the peripheral edges intersect each other), the through-holes 80 are not limited to a mode where a part of the peripheral edge portions 81 forms parts of both peripheral edges of adjacent convexes 31 as in the example illustrated in Fig. 5, and may have a mode where a part of the peripheral edge portions 81 forms a part of a peripheral edge of one convex 31 as illustrated in Fig. 11.

In particular, from a viewpoint of the back-flow, it is preferable that the number of through-holes 80 is small. However, as the number of through-holes 80 decreases, the shape maintaining property of the convexes 31 deteriorates. Therefore, from a viewpoint of achieving both the back-flow and the shape maintaining property of the convexes 31, as illustrated in Fig. 5, it is preferable that the convexes 31 have staggered arrangement, the through-holes 80 are disposed only on both sides in the front-back direction LD at the center of the convex 31 in the width direction WD and both sides in the width direction WD at the center in the front-back direction LD, the peripheral edge portion 81 forms a part of both peripheral edges of the adjacent convexes 31, and the groove 32 extends between adjacent through-holes 80 in a direction along between the adjacent convexes 31.

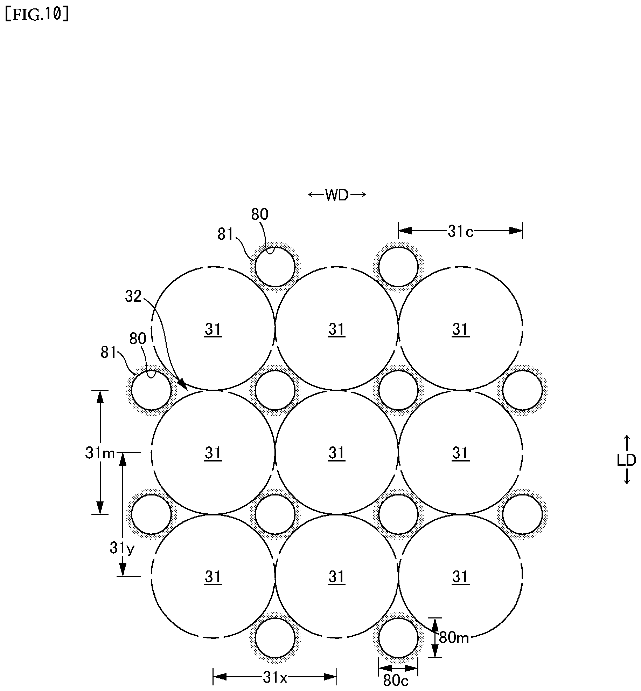

As another arrangement, as illustrated in Fig. 10, the convexes 31 and the through-holes 80 can be disposed in a square lattice or a rectangular lattice.

The shape of the convex 31 is not particularly limited as long as the shape is a dome shape. Here, the term "dome shape" is not limited to a hemispherical shape, and is meant to include a shape having an arbitrary height and an arbitrary planar shape, for example, an approximately elliptical shape or an approximately polygonal shape in plan view.

Even though the dimensions of the convex 31 are not particularly limited, a dimension 31m of the convex 31 in the front-back direction is preferably in a range of 3 to 10 mm (particularly 4 to 7 mm). A dimension 31c of the convex 31 in the width direction is preferably in a range of 3 to 10 mm (particularly 4 to 7 mm). The dimension 31m of the convex 31 in the front-back direction is preferably 0.5 to 2.0 times (particularly 1.1 to 1.5 times) the dimension 31c of the convex 31 in the width direction. In addition, it is preferable that a center interval 31y of the convexes 31 in the front-back direction in a row of the convexes 31 arranged in the front-back direction LD is in a range of 3.5 to 18 mm (particularly 5 to 8 mm). It is preferable that a center interval 31x of the convexes 31 in the width direction in a row of the convexes 31 arranged in the width direction WD is in a range of 3.3 to 16 mm (particularly 4.5 to 7.5 mm). The center interval 31y in the front-back direction is preferably 0.4 to 2.0 times (particularly 0.8 to 1.3 times) the center interval 31x in the width direction. In addition, it is preferable that a height 31z of the convexes 31 is usually set to about 0.7 to 1.8 mm (particularly, 1.0 to 1.5 mm).

In addition, a shape of the through-holes 80 can be determined as appropriate, and can be set to any shape such as a perfect circuit, an ellipse, a polygon such as a triangle, a rectangle, and a rhombus, a star shape, a cloud shape, and the like.

Even though the dimensions of the through-holes 80 are not particularly limited, a dimension 80m of the through-hole 80 in the front-back direction is preferably 5 to 30% (particularly 7 to 14%) of the dimension 31m of the convex 31 in the front-back direction. A dimension 80c of the through-hole 80 in the width direction is preferably 5 to 30% (particularly 7 to 14%) of the dimension 31c of the convex 31 in the width direction. The dimension 80m of the through-hole 80 in the front-back direction is preferably 0.5 to 2.0 times (particularly 0.7 to 1.5 times) the dimension 80c of the through-hole 80 in the width direction.

The convexes 31 and the through-holes 80 may be provided on the entire top sheet 22, or may be provided only in a part in at least one of the front-back direction LD and the width direction WD. For example, the convexes 31 and the through-holes 80 may be provided only in an intermediate portion in the width direction in a surface exposed portion of the top sheet 22.

The top sheet 22 may be bonded to a member on the back surface side by the hot melt adhesive. When the top sheet 22 is bonded to the member on the back surface side by the hot melt adhesive, it is preferable that the hot melt adhesive is intermittently applied by a summit or spiral application or a pattern coat (transfer of the hot melt adhesive in a letterpress method) in a portion overlapping with the absorbers 23A and 23B. On the other hand, it is preferable to solidly apply the hot melt adhesive to portions not overlapping with the absorbers 23A and 23B such as front and back end portions of the top sheet 22 and firmly fix the portions.

### <Description of Terms Used Herein>

In a case where the following terms are used in the specification, those have the following meanings unless otherwise specified in the specification.
· The "front-back (vertical) direction" means a direction connecting the ventral side (front side) and the dorsal side (back side), and the "width direction" means a direction (right-left direction) orthogonal to the front-back direction.
· The "MD (Machine Direction or line flow direction)" and the "CD (Cross Direction) (lateral direction orthogonal to the MD)" in a manufacturing method mean the "MD" and the "CD" of processing equipment of the convexes 31. Either one corresponds to the front-back direction, and the other one corresponds to the width direction. In addition, the MD in the product is a direction of fiber orientation of the nonwoven fabric. Fiber orientation is a direction along which a fiber of a nonwoven fabric runs and determined by, for example, a measurement method in accordance with the fiber orientation test method based on the zero span tensile strength of TAPPI standard method T481 and a simple measurement method for determining the direction of fiber orientation from the ratio of the tensile strength in the front-back direction to the width direction. In the illustrated embodiment, similarly to most disposable diaper products, the front-back direction corresponds to the MD, and the width direction corresponds to the CD.
· "Spread state" means a flatly spread state without contraction or slack.
· "Stretch rate" means the value when the natural length is taken as 100%.
· "Basis weight" is measured as follows. After the sample or test piece is preliminary dried, it is allowed to stand in a test room or apparatus under normal conditions (the test location is at a temperature: 23 ± 1°C, relative humidity: 50 ± 2%) until the constant mass. The preliminary drying is to make the sample or test piece be constant mass in an environment of a temperature of 100°C. Note that the fibers of an official moisture regain of 0.0% do not need preliminary drying. From a test piece having a constant weight, a sample having a size of 100 mm × 100 mm is cut out using a template for sampling (100 mm × 100 mm). The sample is weighed and the weight is multiplied by 100 into the weight per one square meter. The resulting value is defined as the basis weight.
· "Thickness" of the top sheet means "apparent thickness" and is measured by the following method. That is, upon measurement, a measuring piece of 30 mm long × 30 mm wide is cut out. At this time, a cut surface passing through a measurement portion is made. For example, in the case of measuring an apparent thickness of a portion of the top sheet excluding the peripheral edge portions of the through-holes, a cut surface that is parallel to the MD and does not pass through the through-holes and the peripheral edge portions is formed. Then, an enlarged photograph of the cut surface is photographed using a digital microscope VHX-1000 manufactured by KEYENCE CORPORATION, and an apparent thickness of a target portion of the top sheet is measured based on the enlarged photograph. In the case of obtaining the maximum value and minimum value, 10 points or more are measured, and the maximum value and minimum value are measured.
· "Thickness" of the absorber is measured using a thickness measuring instrument of OZAKI MFG. CO., LTD. (PEACOCK, large dial thickness gauge, model J-B (measurement range 0 to 35 mm) or model K-4 (measurement range 0 to 50 mm)) by making the sample and the thickness measuring instrument horizontal.
· "Thickness" other than the above thickness is automatically measured using an automatic thickness measuring instrument (KES-G5 Handy Compression Measurement Program) under the conditions of load: 0.098 N/cm² and pressurized area: 2 cm².
· Water absorption capacity is measured according to JIS K7223-1996 "Testing method for water absorption capacity of super absorbent polymers".
· "Water absorption rate" is the "time that elapses before the end point" measured in accordance with JIS K7224-1996 "Testing method for water absorption rate of super absorbent polymers" has been carried out using 2 g of super absorbent polymer and 50 g of physiological saline solution.
· "Artificial urine" is prepared by mixing urea: 2 wt%, sodium chloride: 0.8 wt%, calcium chloride dihydrate: 0.03 wt%, magnesium sulfate heptahydrate: 0.08 wt%, and ion exchanged water: 97.09 wt%, and those are used at a temperature of 40°C unless otherwise specified.
· When environmental conditions in tests and measurements are not described, the tests and measurements shall be carried out in a test room or apparatus under normal conditions (the test location is at a temperature: 23 ± 1°C, relative humidity: 50 ± 2%).
· The dimension of each part means the dimension in the spread state, not the natural length state, unless otherwise specified.

### Industrial Applicability

The invention can be used in any form, such as a tapetype disposable diaper or an underpants-type disposable diaper in addition to the pad-type disposable diaper, and can also be used for absorbent articles other than disposable diapers, such as sanitary napkins.

### Reference Signs List

- 200: PAD-TYPE DISPOSABLE DIAPER
- 21: LIQUID IMPERVIOUS SHEET
- 22: TOP SHEET
- 22t: THICKNESS
- 23A, 23B: ABSORBER
- 23A: UPPER LAYER ABSORBER
- 23B: LOWER LAYER ABSORBER
- 24: THREE-DIMENSIONAL GATHER
- 24s: GATHER SHEET
- 25: INTERMEDIATE SHEET
- 26: WRAPPING SHEET
- 27: OUTER SHEET
- 30: RECESSED PORTION
- 31: CONVEX
- 32: GROOVE
- 32b: BOTTOM PORTION
- 40: SLIT
- 80: THROUGH-HOLE
- 81: PERIPHERAL EDGE PORTION
- B2: BACK SIDE PORTION
- C2: CROTCH PORTION
- F2: FRONT SIDE PORTION
- LD: FRONT-BACK DIRECTION
- WD: WIDTH DIRECTION

## Claims

1. An absorbent article comprising:
an absorber (23A, 23B); and
a top sheet (22) that covers a front surface side of the absorber (23A, 23B),
the top sheet (22) being formed of a nonwoven fabric,
a plurality of dome-shaped convexes (31) protruding to the front surface side being arranged on the top sheet (22), grooves (32) being formed between adjacent convexes (31),
wherein through-holes (80) penetrating the top sheet (22) in a thickness (22t) direction are provided at intervals in a circumferential direction of each of the convexes (31), the through-holes (80) having front and back sides,
peripheral edge portions (81) of the through-holes (80) are high-density portions where constituent fibers of the nonwoven fabric are pushed to parts around the through-holes (80) from positions corresponding to the through-holes (80),
the high-density portions are not compressed in the thickness direction,
the peripheral edge portions (81) of the through-holes (80) rise to both the front and back sides,
a part of peripheral edges of the convexes (31) is formed by a part of the peripheral edge portions (81) of the through-holes (80), and
a difference between a maximum value and a minimum value of an apparent thickness (22t) is in a range of 0 to 50% of the maximum value in a portion of the top sheet (22) excluding the peripheral edge portions (81) of the through-holes (80).

2. The absorbent article according to claim 1,
wherein a fineness of fibers of the top sheet (22) is in a range of 1.0 to 3.0 dtex, and
a fiber density of the portion of the top sheet (22) excluding the peripheral edge portions (81) of the through-holes (80) is in a range of 16 to 32 g/m³.

3. The absorbent article according to claim 1 or 2,
wherein the convexes (31) have staggered arrangement,
the through-holes (80) are disposed on both sides in a front-back direction (LD) at a center of each of the convexes (31) in a width direction (WD) and both sides in the width direction (WD) at a center thereof in the front-back direction (LD), and each of the peripheral edge portions (81) of the through-holes (80) forms parts of both peripheral edges of adjacent convexes (31), and
each of the grooves (32) extends between adjacent through-holes (80) in a direction along between adjacent convexes (31).

4. The absorbent article according to any one of claims 1 to 3, wherein each of the peripheral edge portions (81) of the through-holes (80) has a weir-shaped portion expanding at least on the front surface side.

5. The absorbent article according to any one of claims 1 to 4,
wherein a dimension of each of the convexes (31) in the front-back direction (LD) is in a range of 3 to 10 mm and a dimension thereof in the width direction (WD) is in a range of 3 to 10 mm, and
a dimension of each of the through-holes (80) in the front-back direction (LD) is in a range of 15 to 35% of a dimension of each of the convexes (31) in the front-back direction (LD), and a dimension of each of the through-holes (80) in the width direction (WD) is in a range of 15 to 35% of a dimension of each of the convexes (31) in the width direction (WD).

## Patentansprüche

1. Absorbierender Artikel, umfassend:
einen Absorber (23A, 23B); und
eine Decklage (22), die eine vordere Oberflächenseite des Absorbers (23A, 23B) bedeckt,
wobei die Decklage (22) aus einem Vliesstoff gebildet ist,
eine Vielzahl von kuppelförmigen Konvexitäten (31), die zur vorderen Oberflächenseite hin vorstehen und auf der Decklage (22) angeordnet sind, wobei zwischen benachbarten Konvexitäten (31) Nuten (32) ausgebildet sind,
wobei Durchgangslöcher (80), die die Decklage (22) in einer Richtung einer Dicke (22t) durchdringen, in Intervallen in einer Umfangsrichtung jeder der Konvexitäten (31) vorgesehen sind, wobei die Durchgangslöcher (80) Vorder- und Rückseiten haben,
periphere Randabschnitte (81) der Durchgangslöcher (80) Abschnitte mit hoher Dichte sind, an denen konstituierende Fasern des Vliesstoffs von Positionen, die den Durchgangslöchern (80) entsprechen, zu Teilen um die Durchgangslöcher (80) herum gedrückt werden,
die Abschnitte mit hoher Dichte in der Richtung der Dicke nicht komprimiert sind,
die peripheren Randabschnitte (81) der Durchgangslöcher (80) sowohl zur Vorderseite als auch zur Rückseite hin ansteigen,
ein Teil von Umfangskanten der Konvexitäten (31) durch einen Teil der peripheren Randabschnitte (81) der Durchgangslöcher (80) gebildet wird, und
eine Differenz zwischen einem Maximalwert und einem Minimalwert einer ersichtlichen Dicke (22t) in einem Bereich von 0 bis 50 % des Maximalwerts in einem Abschnitt der Decklage (22) mit Ausnahme der peripheren Randabschnitte (81) der Durchgangslöcher (80) liegt.

2. Absorbierender Artikel nach Anspruch 1,
wobei eine Feinheit der Fasern der Decklage (22) in einem Bereich von 1,0 bis 3,0 dtex liegt, und
eine Faserdichte des Abschnitts der Decklage (22) mit Ausnahme der peripheren Randabschnitte (81) der Durchgangslöcher (80) in einem Bereich von 16 bis 32 g/m³ liegt.

3. Absorbierender Artikel nach Anspruch 1 oder 2,
wobei die Konvexitäten (31) eine versetzte Anordnung aufweisen,
die Durchgangslöcher (80) auf beiden Seiten in einer Vorderseiten-Rückseiten-Richtung (LD) in einer Mitte jeder der Konvexitäten (31) in einer Breitenrichtung (WD) und auf beiden Seiten in der Breitenrichtung (WD) in einer Mitte davon in der Vorderseiten-Rückseiten-Richtung (LD) angeordnet sind, und jeder der peripheren Randabschnitte (81) der Durchgangslöcher (80) Teile beider peripheren Ränder benachbarter Konvexitäten (31) bildet, und
jede der Nuten (32) sich zwischen benachbarten Durchgangslöchern (80) in einer Richtung entlang zwischen benachbarten Konvexitäten (31) erstreckt.

4. Absorbierender Artikel nach einem der Ansprüche 1 bis 3, wobei jeder der peripheren Randabschnitte (81) der Durchgangslöcher (80) einen wehrförmigen Abschnitt aufweist, der sich zumindest an der vorderen Oberflächenseite ausdehnt.

5. Absorbierender Artikel nach einem der Ansprüche 1 bis 4,
wobei eine Abmessung jeder der Konvexitäten (31) in der Vorderseiten-Rückseiten-Richtung (LD) in einem Bereich von 3 bis 10 mm liegt und eine Abmessung davon in der Breitenrichtung (WD) in einem Bereich von 3 bis 10 mm liegt, und
eine Abmessung jedes der Durchgangslöcher (80) in der Vorderseiten-Rückseiten-Richtung (LD) in einem Bereich von 15 bis 35% einer Abmessung jeder der Konvexitäten (31) in der Vorderseiten-Rückseiten-Richtung (LD) liegt und eine Abmessung jedes der Durchgangslöcher (80) in der Breitenrichtung (WD) in einem Bereich von 15 bis 35% einer Abmessung jeder der Konvexitäten (31) in der Breitenrichtung (WD) liegt.

## Revendications

1. - Article absorbant comprenant :
un absorbant (23A, 23B) ; et
une feuille supérieure (22) qui recouvre un côté de surface avant de l'absorbant (23A, 23B),
la feuille supérieure (22) étant formée d'un tissu non tissé,
plusieurs éléments convexes (31) en forme de dôme faisant saillie sur le côté de surface avant étant disposés sur la feuille supérieure (22), des rainures (32) étant formées entre des éléments convexes adjacents (31),
des trous traversants (80) pénétrant dans la feuille supérieure (22) dans une direction d'épaisseur (22t) étant prévus à intervalles dans une direction périphérique de chacun des éléments convexes (31), les trous traversants (80) ayant des côtés avant et arrière,
les parties de bord périphérique (81) des trous traversants (80) étant des parties de haute densité où les fibres constitutives du tissu non tissé sont poussées vers des parties autour des trous traversants (80) à partir de positions correspondant aux trous traversants (80),
les parties de haute densité n'étant pas comprimées dans la direction d'épaisseur,
les parties de bord périphérique (81) des trous traversants (80) s'élevant à la fois sur les côtés avant et arrière,
une partie des bords périphérique des éléments convexes (31) étant formée par une partie des parties de bord périphérique (81) des trous traversants (80), et
une différence entre une valeur maximale et une valeur minimale d'une épaisseur apparente (22t) étant dans une plage de 0 à 50 % de la valeur maximale dans une partie de la feuille supérieure (22) à l'exclusion des parties de bord périphérique (81) des trous traversants (80).

2. - Article absorbant selon la revendication 1,
dans lequel une finesse des fibres de la feuille supérieure (22) est dans une plage de 1,0 à 3,0 dtex, et
une densité de fibres de la partie de la feuille supérieure (22) à l'exclusion des parties de bord périphérique (81) des trous traversants (80) est dans une plage de 16 à 32 g/m3.

3. - Article absorbant selon l'une des revendications 1 ou 2,
dans lequel les éléments convexes (31) ont une disposition en quinconce,
les trous traversants (80) sont disposés des deux côtés dans une direction avant-arrière (LD) à un centre de chacun des éléments convexes (31) dans une direction de la largeur (WD) et des deux côtés dans la direction de la largeur (WD) à un centre de chacun de ceux-ci dans la direction avant-arrière (LD), et chacune des parties de bord périphérique (81) des trous traversants (80) forme des parties des deux bords périphériques d'éléments convexes adjacents (31), et
chacune des rainures (32) s'étend entre des trous traversants (80) adjacents dans une direction entre des éléments convexes adjacents (31).

4. - Article absorbant selon l'une quelconque des revendications 1 à 3, dans lequel chacune des parties de bord périphérique (81) des trous traversants (80) a une partie en forme de déversoir s'étendant au moins du côté de surface avant.

5. - Article absorbant selon l'une quelconque des revendications 1 à 4,
dans lequel une dimension de chacun des éléments convexes (31) dans la direction avant-arrière (LD) est dans une plage de 3 à 10 mm et une dimension de chacun de ceux-ci dans la direction de la largeur (WD) est dans une plage de 3 à 10 mm, et
une dimension de chacun des trous traversants (80) dans la direction avant-arrière (LD) est dans une plage de 15 à 35 % d'une dimension de chacun des éléments convexes (31) dans la direction avant-arrière (LD), et une dimension de chacun des trous traversants (80) dans la direction de la largeur (WD) est dans une plage de 15 à 35 % d'une dimension de chacun des éléments convexes (31) dans la direction de la largeur (WD).
